Europäisches Patentamt

European Patent Office

Office européen des brevets

(11) Publication number: **0 632 268 A1**

## EUROPEAN PATENT APPLICATION

(21) Application number: **94109933.5**

(22) Date of filing: **28.06.94**

(51) Int. Cl.6: **G01N 33/00**

(30) Priority: **01.07.93 US 86266**

(43) Date of publication of application:
**04.01.95 Bulletin 95/01**

(84) Designated Contracting States:
**DE ES FR GB IT**

(71) Applicant: **Johnson Service Company**
**5757 North Green Bay Avenue**
**Milwaukee**
**Wisconsin 53209 (US)**

(72) Inventor: **Wenger, Jarrell D.**
**5011 N. Berkeley**
**Whitefish Bay,**

**Wisconsin 53217 (US)**
Inventor: **Miller, Russell C.**
**2320 Ronke Lane**
**New Berlin,**
**Wisconsin 53151 (US)**
Inventor: **Ouistgaard, Dorte**
**3424 N. Oakland**
**Milwaukee,**
**Wisconsin 53211 (US)**

(74) Representative: **Bergen, Klaus, Dipl.-Ing. et al**
**Patentanwälte Dr.-Ing. Reimar König,**
**Dipl.-Ing. Klaus Bergen,**
**Wilhelm-Tell-Strasse 14**
**D-40219 Düsseldorf (DE)**

(54) **Apparatus and method for determining the indoor air quality within an enclosed space.**

(57) An instrument (10) for evaluating the quality of air within an enclosed space includes a sensor array (24) and a data processing system (30), the sensor array being positioned to monitor the air within the space, with at least two nonspecific sensors for detecting odiferous and nonodiferous pollutants present in the air, a humidity sensor and sensors for detecting temperature and carbon dioxide, the data processing system including a computer which collects the sensor signal data and processes it to extract features from the same, and being controlled by pattern recognition software to analyze the data to determine the pollution index of the air. The pattern recognition software of choice is an artificial neural network (50) but alternative means include direction cosine or Euclidean distance analysis systems.

FIG. 1

BACKGROUND OF THE INVENTION

The present invention relates generally to the field of environmental control of enclosed spaces, and more specifically to the field of instruments designed to monitor and control interior environments.

Control of interior environments has gone beyond merely addressing the temperature and humidity of the air to attempting to identify and eliminate airborne contaminants as well. Under the rubric of "indoor air quality", such programs have tried to identify the causes of so-called "sick building syndrome" and to take appropriate corrective measures.

A basic problem, however, is the sheer numbers of potential contaminants. As discussed below, sensors are readily available for identifying a number of such compounds, but significant problems have prevented the development and implementation of a workable sensor/control system. Such a system is the goal of the present invention.

It is known in the art to combine sensors with computers in an effort to identify odors. Examples of such systems can be found in journal articles such as the following: Nakamoto, Fukuda & Moriizumi, "Improvement of identification capability in an odor-sensing system," 3 Sensors and Actuators B 221 (1991); Gardner, "Detection of vapours and odours from a multisensor array using pattern recognition", 4 Sensors and Actuators 109 (1991); and Shurmer, "The fifth sense: On the scent of the electronic nose," IEE Review 95 (March 1990). Patents that address this problem include U.S. Patents No. 5,088,314 (Takashi, Feb. 18, 1992); 4,884,435 (Ehara, Dec. 5, 1989); 4,770,027 (Ehara, Sep. 13, 1988); and 4,399,687 (Collins, Aug. 23, 1983). Again, none of these references discloses an apparatus that provides indoor air quality information required for environmental control.

SUMMARY OF THE INVENTION

The broad object of the present invention is to provide an indoor air quality sensor that simulates the olfactory response of a human occupant of an enclosed space.

That and other objects are achieved in the present invention, an instrument for evaluating the quality of air within an enclosed space. A plurality of sensors is positioned to monitor the air within the space, and this sensor array includes at least two nonspecific sensors for detecting odiferous and nonodiferous pollutants present in the air as well as a humidity sensor. It is preferred that the sensor array also include sensors for temperature and carbon dioxide. Each sensor provides an electrical signal proportional to the activation thereof. A computer collects the sensor signal data and processes it to extract features from the same, and pattern recognition software analyzes the data to determine the pollution index of the air. The pattern recognition software of choice is an artificial neural network, but alternative means include direction cosine or Euclidean distance analysis systems.

BRIEF DESCRIPTION OF THE DRAWINGS

FIGURE 1 is a schematic diagram of a preferred embodiment of the present invention;
FIGURE 2 is a schematic diagram of the artificial neural network of the embodiment shown in Fig. 1;
FIGURE 3 is a graphical plot of predicted decipol responses to various enclosed spaces provided by the present invention, against actual human panel responses;
FIGURES 4(a) and (b) are correlation charts showing correlation coefficients plotting against the number of sensors employed.

DETAILED DESCRIPTION OF A PREFERRED EMBODIMENT

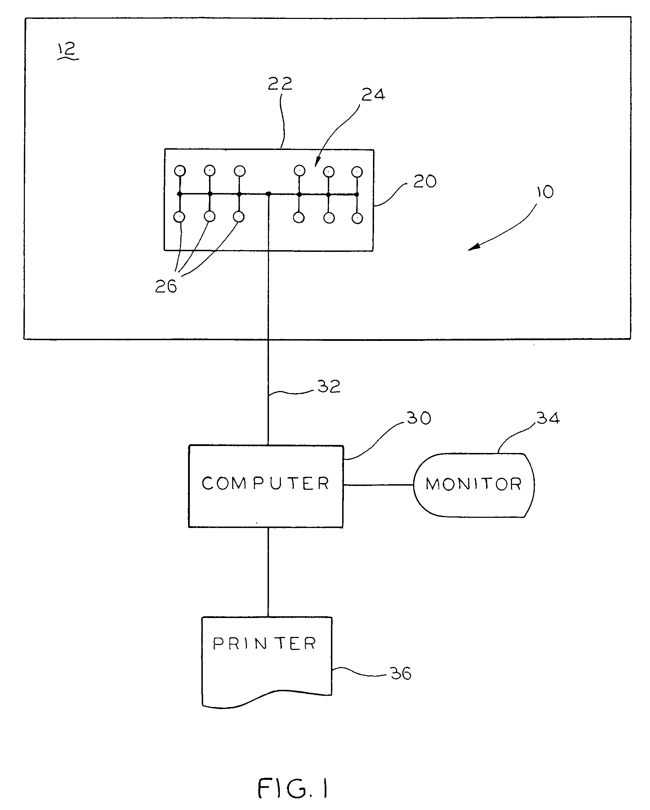

An instrument 10 for monitoring the indoor air quality (IAQ) of an enclosed space 12 is seen in Fig. 1. As is immediately apparent, the device has two major components, a sensor subassembly 20 and computer 30, joined by cable 32. The instrument serves an enclosed space, which can be a room, office, manufacturing area or any other portion of a building selected for environmental control, providing an indication of the overall quality of the air within the space, in terms of a signal that can be monitored by occupants or maintenance personnel, or passed on to a facility control system.

The only portion of the device that must be located in the space is the sensor subassembly, composed of the sensor enclosure 22 and the sensor array 24. The former can take any of the various forms familiar to those in the art for enclosing environmental monitoring instruments. As is typical of such devices, provision must be made for air from the space to make contact with the sensor elements themselves. If desirable, a fan (not shown) can be mounted on the enclosure to promote air flow through the unit.

Similarly, those in the art are capable of analyzing a particular enclosed space to determine optimal locations for positioning the sensor array enclosure to insure exposure to a representative sample of the air. Such techniques do not differ appreciably from those currently employed for disclosing temperature and humidity sensors and the like.

The sensor array itself is made up of sensor elements 26, each of which reacts to a selected environmental variable (or group of variables) and provides and electrical signal output related to the presence of the measured variable. A central problem facing IAQ systems has been the daunting variety of compounds that could contribute to indoor air quality problems. It has been argued that no system could provide sufficient numbers of sensors to detect all of the possible contaminants. The present inventors, however, recognized that problem and looked for a different approach.

The answer lies in the human olfactory system. Consider that humans have only some 30 different olfactory receptors, yet we can recognize hundreds of different substances by their distinctive smells. Clearly, olfactory recognition is based on something other than the stimulation of specific, dedicated receptors for every "known" substance. A more likely explanation depends on the brain's pattern recognition capabilities, coupled with relatively non-specific olfactory receptors. Under that model, a "test" substance would activate a number of receptors to differing degrees, and the brain would interpret the resulting pattern of neural signals as a particular smell.

That is the approach of the present invention. At the threshold, it should be noted that the existing level of knowledge cannot even specify what substances constitute the complete set of compounds that can contaminate a space. Thus, discussion tends to focus on broad groups of potential pollutants, such as "total volatile organic compounds" (TVOC), or "cooking gases" and the like. Moreover, a direct consequence of the broad range of substances involved in IAQ is the fact that generally available sensors tend to suffer from overlap with and interference from other compounds, even when a sensor aims to detect a single substance.

Thus, the sensor elements chosen for the sensor array of the present invention are generally nonspecific, and are selected to provide a broad range of coverage rather than the ability to identify particular compounds with specificity.

Several different technologies offer promise as sources of IAQ sensors. Perhaps the best known is the family of tin oxide sensors known as "Taguchi gas sensors". These devices are widely available in models designed to detect both specific compounds (such as ethanol, hydrogen sulfide, or ammonia) or groups of substances (such as cooking gases, organic vapors, etc.). Techniques for interfacing and powering these sensors are well known in the art. An alternative source of data is infrared (IR) gas analysis, in which an air sample is exposed to pulsed infrared light of narrow bandwidth and a transducer monitors any pressure differential that results from gas molecules absorbing the IR energy. Because gases absorb IR energy only at characteristic wavelengths, the detected pressure change provides a positive indication of the presence of a given gas. Yet another technology uses quartz crystal microbalance sensors, in which a quartz resonator is coated with a layer of gas-absorbing material; presence of the sought-after gas is indicated by a change in the resonant frequency of the crystal. As will be clear from the following discussion, any of these technologies can be employed to provide sensor signals useful in the present invention. The important consideration is to select a sensor array that will provide a broad spectrum of coverage for potential pollutants.

In addition to detecting contaminant gases, it has been found useful to monitor the temperature and humidity within the space. An objective of any IAQ system is to optimize the comfort of occupants of the space, so the utility of such measurements should be clear. Any convenient, available temperature sensor and humidity sensor, such as the combination instrument manufactured by Johnson Controls, Inc. as model HE-6310, can be used for this purpose. If desired, the temperature sensor can be omitted, but it is preferred to include it.

At the present time, none of these technologies has emerged as dominant, and no specific configuration of sensors has been proven optimum. Criteria for choosing the number of sensors is set out in more detail below. It can be said, however, that the sensor array should include temperature and humidity sensors, together with several gas sensors. It is also believed that one of the gas sensors should be a carbon dioxide sensor, as an increased concentration of that gas leads to stuffiness and generally lowered perceived air quality, and is generally a good indicator of increased usage of the space by people. It should also be noted that most existing sensors concentrate on odiferous pollutant compounds. The present invention is not so limited, and sensors to detect nonodiferous contaminants can also be included. Also, biological contaminants could also be detected by sensing gaseous emissions from molds, fungi and the like.

The sensor array employed in the study reported below included a total of 22 sensors of various kinds. Eight Taguchi tin oxide gas sensors were employed, specified as detecting ethanol, hydrogen sulfide, ammonia, cooking gases, organic vapors, combustible gases, air quality and carbon monoxide. In addition, gas analysis sensing was used, with bandwidth filters selected for acetone, carbon dioxide, ethanol, toluene, propane, water vapor, ammonia, vinyl chloride, chloroform, benzene and formaldehyde. A multi-gas analyzer (the Model 1302 Bruel & Kjaer Multi-Gas Monitor) was used for this purpose. The remaining two sensor signals were provided by the temperature/humidity sensor noted above.

Signals from each sensor are fed by cable 32 to computer 30, where it is first processed to extract significant features. Any convenient computer system can be employed; in the embodiment shown here, a personal computer provided the control and analysis, but a facility control system operating at a central location could perform similar services. Output can be provided on a monitor 34 or printer 36. Also, the software can be either specially written for this application or a commercial package can be adapted as necessary. The functional requirements of the software will be described below, and those in the art will be capable of writing or modifying software accordingly.

On arrival at the computer, it is preferred to perform an initial processing step to condition the data for subsequent use. Although a number of data conditioning methods are known, it is preferred to average the data over a sensor reading period, and for the neural network (described below) to normalize the data by scaling each data point to read between a minimum of 0 and a maximum of 1. This treatment insures that succeeding data processing steps do not have to take into account the type of sensor being employed, as every sensor input is converted to a 0-1 reading scale. The averaging step insures that momentary swings in a variable do not influence the result. The averaging time period can be selected to provide desired sensitivity at a given location.

Another threshold issue is the nature of the output, IAQ being a somewhat broad and undefined category. Ultimately, however, IAQ resolves to a human response to conditions within a space, and it was decided to rely on response-based criteria. Pioneering studies in Denmark proposed the "olf" as a standard unit of measure, defined as a the emission rate of bioeffluent air pollutants from a "standard person," which in turn is defined as a sedentary person, in thermal comfort, at an activity level of 1 met, having an average skin area of 1.8 $m^2$, a hygienic level corresponding to 0.7 baths per day and a daily change of underwear. It has been found that this pollution standard can be taught to human observers, who can then relate other air pollution in terms of relative dissatisfaction. To judge the quality of air in a space, the related unit of the "decipol" is proposed, defined as a pollution level of one olf, ventilated by 10 liters/second of unpolluted air.

The objective, simply stated, is to provide an instrument that responds like a human to the odors within a space. In terms of the present device, the objective can be formulated as to provide an output, in decipol, that corresponds to the response of an average human occupant of the space. It has been found that a reliable output indication of IAQ can determined by subjecting the conditioned data to pattern recognition analysis. A variety of pattern recognition techniques are available in the art, but the most useful have been discovered to be an artificial neural network, direction cosine analysis, and a Euclidean distance measure. Each will be described in turn.

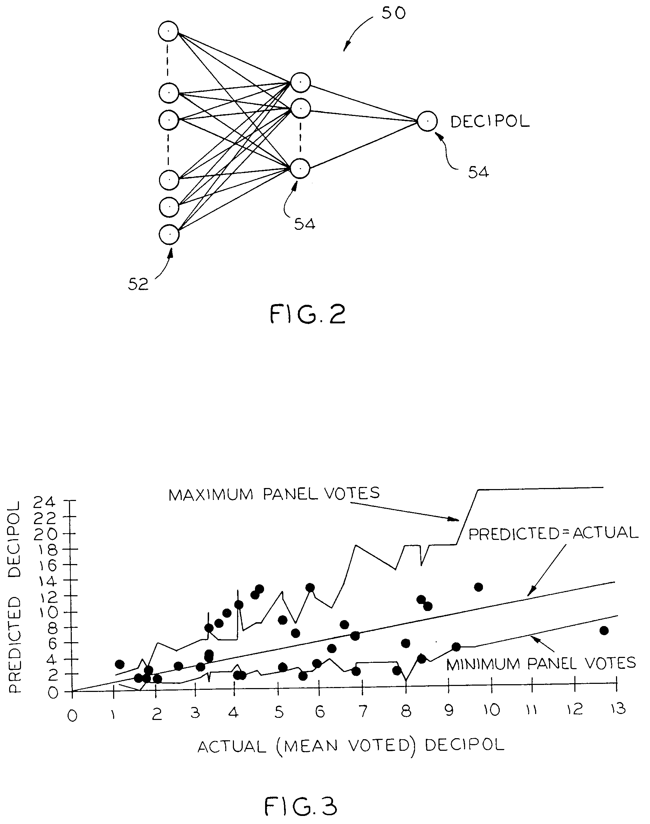

An artificial neural network (ANN) is a collection of simple processing units, interconnected much like brain neurons and synapses. Processing units are characterized by layers, with interconnections between each node and all nodes of the previous and the subsequent layers. Fig. 2 illustrates the ANN 50 used in the present invention, showing the input layer 52, the output layer 54, and the hidden layer 56. It can be seen that each node of the hidden layer is connected to every node of the input layer and to the single node of the output layer. Processing within the nodes of the ANN is conceptually simple, with a node accepting inputs from each interconnection, applying a selected weighing factor to the input, summing the resulting values, and applying an activation function to obtain an output. The power of an ANN is in the assignment of weighing factors, which is controlled during "training" sessions; in broad terms, an ANN "practices" an application, compares its results to some standard, and then adjusts its weighing factors to minimize the error between its results and the standard. By iterative "practice", the ANN "learns" how to solve a problem, under control of its operating algorithm. A successful ANN will become "trained" to the point where it is capable of solving a new problem.

ANN software is thus a generic framework rather than a specific application tool. The control algorithm chosen for the present invention is known in the art as "back propagation" and can be implemented through specially written routines (as in the present invention) or through commercially available ANN software known in the art. Examples of the latter are software packages marketed under the trademarks NEURALWARE and BRAIN MAKER. The ANN 50 is a three-layer network in which the input layer had 22 nodes, the hidden layer 24 nodes and the output layer a single node. The activation function employed was a sigmoid function, as understood in the art. Such a network is readily implemented by those in the art employing either specially-written software or any of the commercial systems noted.

Another pattern recognition technique is direction cosine analysis. This technique analyzes the information contained in the relative magnitudes of information components. For example, if $X = \{x_1, x_2, ..., x_n\}$ are sensor and instrument values for a known sample, and $Y = \{y_1, y_2, ..., y_n\}$ are sensor and instrument values for an unknown sample, then a direction cosine is defined as

$$\cos\theta = \frac{(X, Y)}{\|X\|\|Y\|}$$

The scalar product of X and Y is then defined as

$$(X, Y) = \sum_{i=1}^{n} x_i * y_i$$

and the Euclidean norms of X and Y are defined as

$$\|X\| = \sqrt{\sum_{i=1}^{n} x_i^2}$$

and

$$\|Y\| = \sqrt{\sum_{i=1}^{n} y_i^2}$$

If $\cos \theta = 1$, then the two sets are coincident (i.e., the closer to 1, the better the match between the sets), while if $\cos \theta = 0$, vectors X and Y are orthogonal.

A third measure of similarity is the Euclidean distance, in a multi-dimensional cartesian coordinate system, between X and Y, which can be defined as

$$\rho_E(X, Y) = \sqrt{\sum_{i=1}^{n} (x_i - y_i)^2}$$

This method of similarity is sensitive to the lengths of the vectors unless it is normalized (i.e., X and Y are scaled to constant length); after normalization it is comparable to direction cosines. The closer $\rho_E(X,Y)$ is to zero, the better X and Y match, while a larger distance shows greater dissimilarity.

Each of the pattern recognition techniques requires "training" (in the case of the ANN) or comparison (for the other techniques) in order to adapt it to the particular problem under study. To accomplish this preparation, and to determine whether the invention could successfully replicate human responses to air quality within a space, a panel of human testers was trained in evaluating air quality in terms of decipol values. Then, both the panel and the invention (using all three pattern recognition techniques) tested a set of enclosed spaces. For each tested space, the resulting data set included the sensor readings plus the panel determinations. Next, the data was divided into two subsets, labelled the "training" set and the "test" set. To "train" the ANN, the mean panel result was taken as the "correct" result, and the neural network was exercised as described above, with successive iterations performed to adjust the node weighing factors. Similarly, the cosine direction and Euclidean distance functions were evaluated by assigning the "correct" value of each data set as the panel determination, and calculating to arrive at numerical factors by which future unknown data sets could be characterized. At the end of this process all three pattern recognition techniques were prepared to evaluate new data sets to provide decipol readings.

The "test" data set was then run by each pattern recognition technique, and the result was compared to the human panel determinations. The mean error and standard deviation of error for each method was calculated, and the "best" pattern recognition technique was taken to be the one having the lowest mean error and the lowest standard deviation. Initial results were as follows:

| Technique | Mean Error | SD of Error |
|-----------|-----------|-------------|
| ANN | 2.9 | 2.4 |
| Dir. Cosine | 2.6 | 2.6 |
| Euclidean | 2.2 | 2.9 |

The most important result of these calculations was that the invention did in fact succeed in replicating human response. Fig. 3 plots the decipol predicted by the ANN against the mean panel determination, as well as showing the maximum and minimum values responses of the human testers. As can be seen, the invention provided results very close to the mean decipol, and well within the range of maximum and minimum values. It can safely be stated, then, that the invention provides a realistic and reliable measure of the response of an "average" human occupant of the tested space. It is also noteworthy that the panel standard deviation was 2.9 decipol, so that all methods succeeded in providing an equally consistent or more consistent result than that of the average human observer.

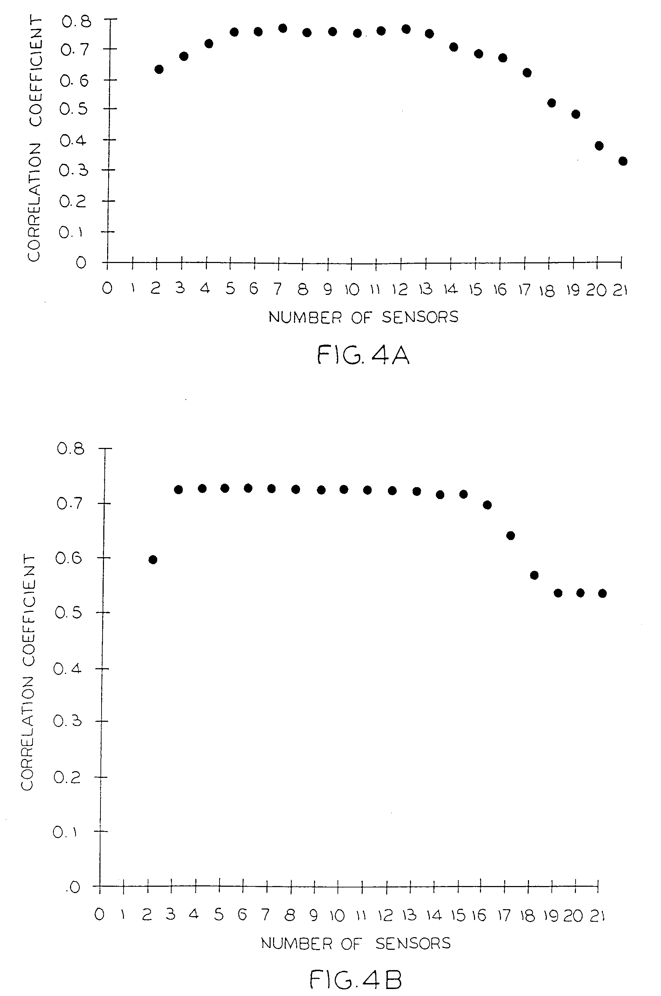

Further analysis of the data was performed to determine whether an optimum number of sensors could be derived. The data were subjected to an analysis that selected "best" subsets of the entire body of sensors and calculated a decipol result based on that subset, using the direction cosine and Euclidean distance methods (the ANN calculation required too much computer time to provide timely results). Figs. 4-(a) and (b) display a correlation chart showing the results of this analysis. As can be seen, both techniques agree that varying the number of sensors produces data that divides into three ranges. Correlation improves with an increased number of sensors for a first range, then plateaus for a second range, and then decreases. Comparing results, it would seem that a minimum of 6-7 sensors would be required for optimum correlation, but after about 14 sensors the "confusion factor" overtakes accuracy, leading to decreased reliability. Selecting an "optimal" 12 sensor set, initial calculations indicate that the cosine direction technique yields a mean error of 1.2 decipol with and 1.4 decipol standard deviation, with a correlation of .77 to the panel determination.

Absolute results were not available for the ANN, but it appears that the ANN achieved a correlation of .79 with panel results.

Those in the art will understand that a variety of changes and modifications to the illustrated embodiment can be made without departing from the spirit of the invention. For example, the exact number and composition of the sensors can be varied, and the makeup of the sensor enclosure can be altered to suit particular environments. These and other changes can be made within the scope of the invention, which is defined solely by the claims appended hereto.

**Claims**

1. An instrument (10) for evaluating the quality of air within an enclosed space, including a plurality of sensors (26) positioned to monitor the air within the space, each of said sensors providing an electrical signal related to the activation thereof; and processing means (30) for collecting said sensor signal data and processing said sensor signal data to extract features from the same, characterised by said plurality of sensors including at least two nonspecific sensors for detecting odiferous and nonodiferous pollutants present in the air, and a humidity sensor, and by pattern recognition means (50) operatively connected to said processing means for determining the pollution index of the air.

2. The instrument of Claim 1, wherein said pattern recognition means is a computer system operating a neural network trained to provide a response equivalent to the response of a human occupant of the space.

3. The instrument of Claim 1, wherein said pattern recognition means is a computer system employing direction cosine analysis to provide a response equivalent to the response of a human occupant of the space.

4. The instrument of Claim 1, wherein said pattern recognition means is a computer system employing Euclidean distance analysis to provide a response equivalent to the response of a human occupant of the space.

5. The instrument of Claim 1, wherein said plurality of sensors further includes a carbon dioxide sensor.

6. The instrument of Claim 1, wherein said plurality of sensors further includes a temperature sensor.

7. An instrument (10) for evaluating the quality of air within an enclosed space, comprising: a plurality of sensors (26) positioned to monitor the air within the space, each said sensor providing an electrical signal related to the activation thereof and a computer system (30) operatively connected to said plurality of sensors for collecting and processing sensor signal data characterised by said plurality of sensors including at least two nonspecific sensors for detecting odiferous and nonodiferous pollutants present in the air, a carbon dioxide sensor, a temperature sensor, and a humidity sensor, and by said computer system including means for collecting, averaging and normalizing said sensor signal data to extract features from the same; and a neural network (50) connected to said collecting, averaging and normalizing means, trained to provide a response equivalent to the response of a human occupant of the space, whereby said computer system provides as output the pollution index of the air within the space.

FIG. 1

FIG. 2

FIG. 3

FIG. 4A

FIG.4B

European Patent Office

# EUROPEAN SEARCH REPORT

Application Number

EP 94 10 9933

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| Y | IEE PROCEEDINGS G. ELECTRONIC CIRCUITS & SYSTEMS, vol.137, no.3, June 1990, STEVENAGE GB pages 197 - 204, XP125837 H. V. SHURMER * page 198, left column, line 1 - line 2 * * paragraph 7; figure 16 * --- | 1-7 | G01N33/00 |
| Y | DE-A-38 26 263 (SIEMENS AG) * abstract * --- | 1-7 | |
| Y | ANALYTICAL CHEMISTRY, vol.58, no.4, April 1986, WASHINGTON, DC, US pages 860 - 866 J. R. STETTER ET AL. * page 862, left column * --- | 3,4 | |
| A | SENSORS AND ACTUATORS B, vol.B9, no.1, July 1992, LAUSANNE CH pages 9 - 15, XP297731 J. W. GARDNER ET AL. * figures 1,3 * --- | 1,2,7 | |
| A | TRAC, TRENDS IN ANALYTICAL CHEMISTRY, vol.11, no.2, February 1992, AMSTERDAM, NL pages 61 - 67, XP247333 K. C. PERSAUD * page 64, right column - page 66, right column * --- | 1,2,7 | TECHNICAL FIELDS SEARCHED (Int.Cl.6) G01N |
| A | ARCHIV FUR ELEKTRONIK UND UBERTRAGUNGSTECHNIK, vol.42, no.2, March 1988, STUTTGART DE pages 85 - 90 G. HORNER ET AL. * the whole document * --- | 1,7 | |

-/--

The present search report has been drawn up for all claims

| Place of search | Date of completion of the search | Examiner |
|---|---|---|
| BERLIN | 14 October 1994 | Brison, O |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
......................................................................
& : member of the same patent family, corresponding
document

EPO FORM 1503 03.82 (P04C01)

European Patent Office

**EUROPEAN SEARCH REPORT**

Application Number

EP 94 10 9933

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A,D | SENSORS AND ACTUATORS B, vol.4, 1991, LAUSANNE CH pages 109 - 115 J. W. GARDNER * the whole document * | 1,7 | |

TECHNICAL FIELDS SEARCHED (Int.Cl.6)

The present search report has been drawn up for all claims

| Place of search | Date of completion of the search | Examiner |
|---|---|---|
| BERLIN | 14 October 1994 | Brison, O |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                             

& : member of the same patent family, corresponding document

EPO FORM 1503 03.82 (P04C01)